Europäisches Patentamt

European Patent Office

Office européen des brevets

(19)

(11) Publication number: **0 114 589**
**B1**

(12) **EUROPEAN PATENT SPECIFICATION**

(45) Date of publication of patent specification: **23.09.87**

(21) Application number: **83870132.4**

(22) Date of filing: **19.12.83**

(51) Int. Cl.⁴: **A 61 K 31/725** //
**(A61K31/725, 31:57)**

(54) Inhibition of angiogenesis.

(30) Priority: **20.12.82 US 451431**
**07.12.83 US 559175**

(43) Date of publication of application:
**01.08.84 Bulletin 84/31**

(45) Publication of the grant of the patent:
**23.09.87 Bulletin 87/39**

(84) Designated Contracting States:
**AT BE CH DE FR GB IT LI LU NL SE**

(56) References cited:
**CHEMICAL ABSTRACTS, vol. 99, no. 15,
October 10, 1983, page 37, ref. 115693q
Columbus, Ohio, US J. FOLKMAN et al.:**

**"Angiogenesis inhibition and tumor regression
caused by heparin or a heparin fragment in the
presence of cortisone"**

**DICTIONNAIRE "VIDAL", 03-02-1961, Office de
Vulgarisation Pharmaceutique, pages 793,
1637 Paris, FR**

(73) Proprietor: **PRESIDENT AND FELLOWS OF
HARVARD COLLEGE
17 Quincy Street
Cambridge Massachusetts 02138 (US)**

(72) Inventor: **Folkman, Moses Judah
18 Chatham Circle
Brookline Massachusetts 02146 (US)**
Inventor: **Taylor, Stephanie
400 Brookline Avenue
Boston Massachusetts 02115 (US)**
Inventor: **Langer, Robert Samuel
46 Greenville Street
Somerville Massachusetts 02143 (US)**

(74) Representative: **Lunt, John Cooper et al
Monsanto Europe S.A. Patent Department
Avenue de Tervuren 270-272 Letter Box No 1
B-1150 Brussels (BE)**

(56) References cited:
**R. BUCHER: DRUGS MADE IN GERMANY, vol.
5, December 1962, pages 201-264 Editio
Cantor, Aulendorf, DE**

**ROTE LISTE, 02-03-1961, Editio Cantor,
Aulendorf/Württ, DE Page 947, "Thrombotison
Dr. Sasse"**

## Description

This invention relates to compositions for the inhibition of angiogenesis and pertains more specifically to compositions for the treatment of mammals with heparin or heparin fragments and with cortisone, hydrocortisone or the 11-α isomer of hydrocortisone to inhibit angiogenesis with subsequent regression of large tumor masses and prevention of tumor metastasis in mammals containing such tumors.

Angiogenesis, the growth of new capillary blood vessels, is important in normal processes such as development of the embryo, formation of the corpus luteum and wound healing. It is also a component in pathologic processes such as chronic inflammation, certain immune responses and neoplasia. Furthermore, angiogenesis is a property of most solid tumors and is necessary for their continued growth.

It has previously been reported that heparin enhanced the intensity of angiogenesis induced by tumors *in vivo*, although in the absence of tumor cells or tumor extract neither heparin nor the mast cells which release heparin could induce angiogenesis. Taylor and Folkman, Nature Vol. 297, 307—312 (1982). It has also been reported in Shubik et al., J. Natl. Cancer Ist., Vol. 57, 769—774 (1976) that 6 α-methyl-prednisolone partially suppressed tumor angiogenesis in hamster cheek pouch under certain conditions, but tumor growth was not stopped, and many other publications have reported continued growth of tumors even in the presence of large doses of cortisone. It has also been reported in Gross et al., Proc. Natl. Acad. Sci., USA, Vol. 78, 1176—80 (1981) that medroxyprogesterone, dexamethasone, and to a lesser extent cortisone, inhibited tumor angiogenesis in rabbit corneas, while estradiol and testosterone were ineffective.

Heparin, an α,β-glycosidically linked highly sulfated copolymer of uronic acid and glucosamine, has been used clinically as an anticoagulant for half a century. Despite its importance and widespread use, both the exact structure of heparin and the precise nature by which it acts in blood anticoagulation have not been elucidated. Much of the difficulty in determining the structure of heparin is because it is not a homogeneous substance. Heparin is polydisperse with a molecular weight range from 5,000 to 40,000. Within a given chain, there are also structural variations such as varying degrees of sulfation, N-acetylation, and C-5 epimerization in the uronic acid residue.

Consequently, the precise composition of commercial heparin varies depending on its source and method of purification. Heparin has been degraded by treatment with heparinese (an enzyme of bacterial origin, Langer et al. U.S. Patent 4,341,869) which cleaves the molecule at the α-glycosidic linkages between N-sulfated-D-glucosamine 6-sulfate and L-iduronic acid 2-sulfate to form fragments including disaccharide, tetrasaccharide, hexasaccharide, and larger oligosaccharides, each being simply a chain-shortened heparin fragment with minor end group modification (the degradation results in a Δ-4,5 site of unsaturation in the terminal uronic acid residue). Linhardt et al., J. Biol. Chem., Vol. 257, 7310—13 (1982).

Certain compositions for topical application and containing both heparin and hydrocortisone or hydrocortisone acetate are disclosed in the prior art. For example, Dictionnaire VIDAL, 1961 pages 793 and 1637 mentions a pommade and suppositories containing these components together with other ingredients such as methyl or propyl 1,4-oxybenzoate; Drugs Made in Germany, Vol 5, page 201, December 1962 mentions a salve containing hydrocortisone acetate, heparin and neomycin sulphate; and Rote Liste 1961, page 947 mentions an ointment containing heparin, hydrocortisone, and other ingredients including hexachlorophene. The indications stated for these various preparations include the treatment of skin ulcers, eczema, hemorrhoids, thromophlebitis, dermatoses, allergies and inflammatory conditions. None of these preparations is intended for, or would be suitable for, oral or subcutaneous administration.

The invention relates to a composition suitable for oral or subcutaneous administration, for inhibition of angiogenesis in mammals, in which the active agents consist essentially of 1) heparin or a heparin fragment which is a hexasaccharide or larger and 2) cortisone or hydrocortisone or the 11-alpha -isomer of hydrocortisone.

It has now been found that angiogenesis in mammals is inhibited, and tumor masses in mammals are caused to regress (and metastasis prevented), by oral or subcutaneous administration of heparin or heparin fragments which are hexasaccharides or larger together with cortisone or hydrocortisone or the 11-α isomer of hydrocortisone. The full name of the isomer is 11-α, 17,21-trihydroxypregn-4-ene-3,20-dione. Neither mature non-growing blood vessels nor vascular tissue seems to be affected by the procedure of the present invention. Inhibition of angiogenesis in accordance with the present invention, in addition to its effect upon tumor regression and metastasis in tumor-bearing mammals, is effective as a contraceptive in females even if first administered after insemination has occurred, is effective to reduce osteoporosis, and is effective in treating diseases involving neovascularization such as neovascular diseases of the eye.

Neither cortisone nor hydrocortisone nor the 11-α isomer of hydrocortisone effectively inhibits angiogenesis nor causes regression of tumors in the absence of heparin or a heparin fragment. Heparin alone does not inhibit angiogenesis but on the contrary enhances it.

The administration of a composition of the invention may be oral or subcutaneous, "subcutaneous", being understood to include intravenous, intra-arterial or subcutaneous injection. In the case of heparin, which is commercially available in the form of heparin sodium, oral administration leads to degradation in the gastrointestinal tract which results in loss of its anticoagulant activity, but because it has been found that the degradation products include disaccharide and larger fragments, this mode of administration is highly effective for the present invention both for heparin and for heparin fragments. The heparin and its fragments may be employed in any physiologically acceptable non-toxic form, including their metal salts, preferably as the sodium salts, all of which are embraced in the term "heparin" or "fragment" as used in the present specification and claims. For best results heparin sold under the trade name "Panheprin" (Abbott Laboratories) is preferred, but heparin from other sources, such as Hepar, Inc. can also be used although less effective. Cortisone and its physiologically acceptable non-toxic salts, such as the acetate, are only very slightly soluble in water, hence are preferably administered subcutaneously, not orally. For oral administration, hydrocortisone or its 11-α isomer (which are relatively water soluble as compared to cortisone) or one of their water-soluble physiologically acceptable non-toxic salts such as the phosphate are preferred. Water insoluble salts of hydrocortisone or its 11-α isomer which are non-toxic and physiologically acceptable, are administered subcutaneously. The terms "cortisone" and "hydrocortisone" and 11-α isomer of hydrocortisone as used in the present specification and claims are intended to include both the steroids themselves and their salts as defined above.

Dosages employed are limited only by the well known limits for the administration of the drugs individually for their usual effects, in the case of cortisone, hydrocortisone, of its 11-α isomer. Heparin may be administered subcutaneously in amounts as large as tolerable without objectionable anticoagulant effects. Since heparin administered orally has no anticoagulant effect, and since the hexasacharide fragment has no anticoagulant effect whether given orally or in any other way, large dosages can thus be administered without risk of bleeding. Oral dosages of heparin of the order of 27,000—45,000 units per kg body weight per day have been found to be effective, but when administered subcutaneously, doses greater than about 600 units per kg body weight twice daily led to undesirable anticoagulation effects. In the case of the hexasaccharide fragment, 7 mg per kg body weight twice daily subcutaneously has been found effective. Cortisone acetate was effective in subcutaneous dosages of 250 mg/kg/day down to 37 mg/kg/day and hydrocortisone was effective orally in amounts of 0.45 mg/ml drinking water (approximately 75 mg/kg/day). The 11-α isomer of hydrocortisone is approximately equal to hydrocortisone in activity for the purpose of the present invention.

The dose size required to bring about regression of tumors or to prevent metastasis varies to some extent depending upon the identity of the tumor, as does the length of time required to bring about complete regression of tumors. Tumor size at the beginning of treatment also affects the length of time required for complete regression. Because of the occurrence of angiogenesis in psoriasis and arithritis, it is expected that the present invention may be useful in treating these diseases. Because administration of cortisone, with or without heparin or heparin fragments, may result in pulmonary infection after a number of days, it is desirable to administer a suitable antibiotic as prophylaxis during treatment in accordance with the present invention.

The heparin (or fragment) and cortisone or hydrocortisone or the 11-α isomer of hydrocortisone are best dissolved or suspended in a suitable carrier which itself must be non-toxic and physiologically acceptable, such as water or normal saline. Compositions containing mixtures of the active agents, either dry or in a suitable carrier, can be employed.

Example 1

Cortisone acetate 0.9 mg in 0.9 ml saline was flooded over the chorioallantoic membrane of 8-day chick embryos through a window in the shell made previously. On day 9, tumor extract (100μg) from hepatoma cells (as described in Zetter, Nature Vol. 285, 41—43, 1980) in 5 μl $H_2O$ was placed on the center of a round 15 mm diameter plastic coverslip and allowed to dry. To the center of each coverslip was then added a 5 μl aliquot containing either heparin (6 μg, i.e., 1 unit), or water, at least twenty embryos being subjected to each. After drying, the coverslip was placed on the chorioallantoic membrane. Additional control embryos received tumor extract and/or heparin, but were not pre-treated with cortisone acetate. The membranes were viewed on day 11 with a x12 stereoscope. Angiogenesis was present if new capillaries were seen to converge on the spot in the center of the coverslip. All of the embryos treated with water or heparin but no cortisone exhibited angiogenesis, as well as 80% of those treated with cortisone acetate alone. Less than 2% of those treated with both heparin and cortisone acetate exhibited angiogenesis.

Example 2

Porcine mucosal heparin was exhaustively degraded using heparinase by the procedure of Langer et al., Science Vol. 217, 261—3 (1982) and the products were fractionated using Sephadex® columns equilibrated with 1M $NH_4OAc$. The degraded heparin had no anticoagulant activity as determined by activated partial thromboplastic time or whole blood recalcification time. The product or product mixture (250 mg) was dissolved in 1 ml of 1M $NH_4OAc$, loaded onto a 75×2.5 ml G-15 column, and eluted at 0.5 ml/min. This resulted in several incompletely resolved peaks corresponding to tetra- hexa-, and higher oligosaccharides and a separate peak corresponding to disaccharide product. The disaccharide peak was freeze-dried, dissolved in 0.2 ml of 1M $NH_4OAc$ and rechromatographed on G-15 resulting in the same sharp peak which was freeze-dried. The mixture of tetra-, hexa-, and oligosaccharide was freeze-dried, redissolved in 1 ml of 1M $NH_4OAc$, and eluted from a 50×1.25 cm G-50 at 2 ml/min, resulting in an unresolved double peak corresponding to tetra- and hexasaccharide fragments and an additional peak corresponding to oligosaccharides which was freeze-dried. The tetra- and hexasaccharide fragments were

3

combined, freeze-dried, redissolved in 1 ml of 1M NH$_4$OAc, and loaded onto a G-15 column. The tetrasaccharide was eluted from a G-15 column in a single peak, the center cut of which was freeze-dried. The hexasaccharide fraction was freeze-dried, redissolved in 0.3 ml 1M NH$_4$OAc, eluted from a G-15 column in a single peak, the center cut of which was freeze-dried.

Fragment size was determined by dissolving a weighed amount of each fraction into 0.03M hydrochloric acid and measuring the absorbance of this solution at 232 nm. The molecular weight of each fragment was calculated using a molar absorptivity, for the α,β unsaturated carboxylate end group present in each of these products, of ε=5500. The di- and tetrasaccharides were further characterized by comparing the K avg-values on G-15 with mono-, di-, and trisaccharide standards. Measured molecular weights were 530, 1210, 1600, and 1870 for the di-, tetra-, hexa-, and oligosaccharide fractions respectively.

The various heparin fragments were dissolved into methylcellulose discs either alone or with cortisone acetate. The discs were then applied to the 4-day yolk sac membrane of chick embryos cultured in Petri dishes as described by Taylor and Folkman, Nature, Vol. 297, 307—312 (1982). In the presence of cortisone acetate (100 μg), as shown in the following table, the hexasaccharide fragment demonstrated the highest antiangiogenesis activity.

TABLE I
Percent Embryos that developed avascular zones 48 hours after implantation of methylcellulose discs on the 4-day old yolk sac membrane

| Conc. (μg) | Oligo-saccharides | Hexa-saccharide | Tetra-saccharide | Di-saccharide |
|---|---|---|---|---|
| 12 | all died | 100% | 0% | 0% |
| 8 | 100% | 100% | | |
| 4 | 25% | 100% | | |
| 1 | 25% | 50% | | |
| 0.1 | 0 | 50% | | |

Tetra- and disaccharides were inactive. Oligosaccharides were less active and were toxic at higher concentrations. Therefore, the hexasaccharide fragment was used in subsequent experiments. In the growing 6-day chorioallantoic membrane, discs containing hexasaccharide (12 μg) and cortisone acetate (100 μg) produced large avascular zones up to 12.6±0.1 mm diameter by 48 hours. As in the case of heparin (with cortisone acetate), capillaries in the mesodermal layer were absent while the other two tissue layers of the membrane were intact and viable. Hexasaccharide alone did not promote tumor angiogenesis as heparin did.

All discs contained a combination of cortisone acetate (100 μg) and a heparin fragment. No avascular zones developed in the presence of any heparin fragment alone, or with cortisone or methylcellulose alone., Ten embryos were used for each group. With hexasaccharide (plus cortisone), the area of the avascular zone was 17% of the vascular membrane at 12 μg and 15% at 0.1 μg. For the oligosaccharides, the maximum avascular area was 10%.

Example 3
Fertilized chick embryos were removed from their shell on day 3 (or 4) and incubated in a Petri dish in high humidity and 5% CO$_2$ as previously described by Auerbach et al., J. Devel. Biol., Vol. 41, 391-4 (1974), except that an outer dish and antiobiotics were not used. On day 6, a methylcellulose disc (10 μl) containing either heparin (6 μg), or hexasaccharide heparin fragment (12 μg), of cortisone acetate (Sigma, powder free of preservatives and suspending agents), or a combination of cortisone acetate+heparin or cortisone acetate+hexasaccharide was implanted on the chorioallantoic membrane. The embryos were examined 48 hours later, and if a clear avascular zone appeared around the methylcellulose disc, the diameter of the zone was measured with a Nikon® profile projector at x20. Thirty embryos were used in each group. India ink was injected into the heart of some embryos just before formalin fixation of that vessels could be followed to the edge of the avascular zone in histological sections.

Hexasaccharide+cortisone acetate produced avascular zones of 12.6±0.1 mm diameter in all embryos. Heparin+cortisone acetate produced avascular zones of 8.9±0.7 mm diameter. There were no avascular zones in the presence of any compounds alone, or with methylcellulose alone.

Histologic cross-sections of the chorioallantoic membranes, revealed that capillaries developed normally in the presence of any compound alone. In contrast, capillaries were completely absent from the mesodermal layer in the face of either hexasaccharide+cortisone acetate, or heparin+cortisone acetate, while the ectodermal and endodermal cell layers remained unaffected. In the mature chorioallantoic membrane where vessels are no longer growing, the cortisone acetate-heparin or -hexasaccharide fragment combinations were without effect.

Example 4

Polymer pellets of ethylene vinyl acetate copolymer (EVA) of approximately 1 mm diameter were impregnated, using the procedure of Langer et al., Nature, Vol. 263, 797—800 (1976), with heparin 180 μg (Sigma), or hexasaccharide fragment 300 μg, or cortisone acetate 1.5 mg (Sigma), or a combination of cortisone and heparin. The pellets were implanted in the cornea of a rabbit eye 1 mm from the limbus and a 1 mm³ piece of V2 carcinoma was implanted distal to the polymer, 2 mm from the limbus. In the opposite eye of each rabbit, control pellets that were empty were similarly implanted in juxtaposition to the tumor.

Release rates averaged 15 μg/day for heparin; 21 μg/day for hexasaccharide fragment; and 5 μg/day for cortisone. When the compounds were mixed, they released at the same rates. By spectrophotometry, the pellets released heparin for 14 days, hexasaccharide for 11 days, and cortisone for more than 30 days.

As capillary blood vessels grew towards the tumor implant, maximum vessel length was measured every 3 days with a stereoscopic slit lamp at x10 (±0.1 mm). On day 14 the rabbits were killed, and India ink was injected into each carotid artery. The corneas were removed and examined with a stereoscope.

New capillary blood vessels were observed growing towards the tumor and passing over an empty pellet or a pellet containing heparin alone at a mean rate of 0.44 mm/days and over a pellet containing cortisone alone at 0.22 mm/day. The tumors behind these pellets were vascularized by 6—8 days. When the pellets contained both cortisone and heparin, there was no capillary growth for 13 days. When the heparin-cortisone pellets were removed or when the pellets were depleted of heparin, capillary growth resumed. Histologic sections showed that tumor cells remained viable and capable of replication even when they were adjacent to the heparin-cortisone pellet.

In the presence of implanted pellets in which the hexasaccharide fragment of Example 2 replaced the heparin, new capillaries grew toward the tumors at a mean rate of 0.30 mm/day in the presence of the hexasaccharide pellets; 0.14 mm/day when the pellets contained cortisone; and 0.32 mm/day when the pellets were empty. In the presence of the hexasaccharide-cortisone combination, there was no capillary growth throughout the 13-day observation period in 4 rabbits, and in one rabbit a few capillaries grew at 0.07 mm/day.

Example 5

(a) Ovarian Sarcoma: 1 mm³ pieces of tumor were implanted with a trocar subcutaneously in the backs of 30 mice; 5 per group. Treatment was begun 10 days later, when mean tumor volume was $1.5 \times 10^2$ mm³. Oral heparin was 200 U/ml in drinking water, average daily consumption was 3—5 ml water per 22 g mouse. Cortisone acetate was administered subcutaneously once daily in a dose of 250 mg/kg for six days, 125 mg/kg for one day, 75 mg/kg for one day, and thereafter a daily maintenance dose of 37 mg/kg (a "tapered" dosage). Control animals received either saline injections, or heparin alone or cortisone acetate alone. All controls were dead by day 34 with large primary tumors and lung metastases. All mice treated with oral heparin+cortisone tapered dosage became tumor-free by day 15 and remained so after treatment was discontinued.

An additional group of mice was treated similarly except that heparin was administered twice daily subcutaneously in a dose of 627 units, and cortisone acetate was administered subcutaneously once daily in a uniform dose of 75 mg/kg. Response of the mice was the same as in the first set except that tumors recurred after cessation of treatment; these mice became permanently tumor-free when subjected to the regimen of oral heparin and cortisone acetate tapered dosage described above. One of these mice died on day 31 with no gross primary tumor and no metastases.

(b) Lewis Lung Carcinoma: Treatment began 7 days after implantation of a 1 mm³ piece of tumor in 42 mice: 7 per group. Treatment was with oral heparin and subcutaneous cortisone acetate tapered dosages described above. All controls died by day 33 with large tumors and numerous lung metastases. In the heparin+cortisone acetate groups treatment was discontinued for each mouse after tumor had been invisible for approximately 7 days. In the oral heparin+cortisone acetate all mice were off treatment by day 33, and remained tumor-free. In an additional group treated with subcutaneous heparin and subcutaneous cortisone acetate (75 mg/kg), 5 mice were off treatment at day 37 and remained tumor-free. Two mice died of pneumonia on days 30 and 33 respectively with small primary tumors (<75 mm³). One metastasis was found in one mouse.

To determine if other steroids could substitute for cortisone, heparin was administered with hydrocortisone, dexamethasone, or medroxyprogesterone. Only hydrocortisone was as effective as cortisone acetate in causing tumor regression when administered with heparin. At the highest tolerable doses neither dexamethasone (3.2 mg/Kg), nor medroxyprogesterone (112 mg/Kg), caused regression of Lewis lung tumors with or without heparin.

(c) B-16 Melanoma: $7.4 \times 10^6$ melanoma cells were injected subcutaneously into 40 mice; 5 per group. Treatment of one group was with oral heparin as described above and oral hydrocortisone, 0.45 mg/ml in drinking water. Another group received oral heparin and subcutaneous cortisone acetate tapered dosage, and a third group received subcutaneous heparin and cortisone acetate 75 mg/kg. Controls received either water, or heparin alone, or hydrocortisone or cortisone acetate alone. All control animals died by day 31 with large tumors and lung metastases. In the heparin+cortisone acetate groups, treatment was discontinued after tumor had become invisible for approximately 7 days. In the oral heparin+cortisone acetate tapered dosage group, 1 mouse died on day 24 with a partially regressed tumor and 2 lung metastases that were avascular and measured less than 0.1 mm. All other mice in the group became tumor-free and remained so after their treatment was discontinued by day 32. In the subcutaneous heparin+cortisone acetate group one mouse died on day 18 and another on day 21; neither had lung metastases. Treatment was discontinued for the other 3 mice on day 32; tumors 3 weeks later were successfully re-treated with oral heparin+cortisone acetate tapered dosage, and these mice have remained tumor-free. In the oral heparin+oral hydrocortisone group, all mice remained tumor-free after their treatment was discontinued on day 47. The regimen of oral heparin+oral hydrocortisone seemed to be more effective for melanoma than it was for ovarian sarcoma or Lewis lung carcinoma.

(d) Bladder Carcinoma: 70 mice: 7 per group received a 1 mm³ implant of tumor subcutaneously. All control animals died by day 31, with large primary tumors. No mice bearing bladder carcinoma developed lung metastases. One group was treated with oral heparin and subcutaneous cortisone acetate tapered dosage starting on day 9 when mean tumor volume was 140 mm³. Tumors stopped growing, but regressed only partially, and then reached a steady state where tumor volume remained at approximately 70 mm³ for as long as the treatment was continued (i.e., 61 more days). Only one mouse died of pneumonia on day 19. The "dormant" tumors were visable, as evidenced by resumption of tumor growth whenever treatment was discontinued for one mouse at a time, beginning at day 70. For a second group treated with subcutaneous heparin and subcutaneous cortisone acetate 75 mg/kg as described above, the result was similar; i.e., long-term tumor "dormancy". One mouse died at day 21.

Because of the inability the standard regimen of oral heparin+cortisone acetate to bring about complete regression, higher concentrations of oral heparin were used with other groups. With oral heparin (600 U/ml)-cortisone acetate tapered dosage, there was more significant tumor regression and a steady state ("dormancy") was reached at a smaller tumor volume of approximately 45 mm³. One mouse died. However, with 1000 U/ml heparin, there was complete regression; mice remained tumor-free after discontinuation of treatment on day 39. No mice died in this group.

In summary, all tumors either stopped growing or regressed when the heparin-cortisone acetate combination was administered. In contrast, when either compound was used alone, tumor growth continued at the same rate as in animals receiving only saline injections; all such control animals died with a large tumor burden.

In the majority of animals treated with heparin+cortisone acetate, it was possible to achieve "complete regression", i.e., tumors did not recur after treatment was discontinued. Thus, with the most effective regimen, oral heparin (200 U/ml)+cortisone (s.c.—tapered dosage), it was possible to obtain "complete regresssion" in 100% of ovarian sarcomas, 100% of Lewis lung carcinomas and 80% of B-16 melanomas. However, when bladder carcinomas were treated with this regimen, there were no "complete regressions" until heparin was increased to 1000 U/ml, following which 100% of tumors regressed without recurrence. With the less effective regimen, heparin (s.c.)+cortisone (75 mgm), complete regression rate was ovarian, 80%; Lewis lung, 71%; B-16 melanoma 60%; and bladder 0%.

Example 6

The hexasaccharide heparin fragment of Example 2 was dissolved in saline, 1.5 mg/ml. Three mice bearing implanted ovarian sarcoma were treated with subcutaneous injection of the hexasaccharide fragment twice daily at a dosage of 7 mg/kg and subcutaneous injection of cortisone acetate tapered dosage. Control mice received either cortisone acetate alone or saline. While control tumors grew progressively, the hexasaccharide+cortisone acetate treated tumors regressed rapidly and were barely visible 4 days later. Their treatment with hexasaccharide was then discontinued, and the tumors reappeared 3—5 days later.

Example 7

To directly observe avascular tumors during systemic therapy, Lewis lung tumors were implanted in the mouse cornea by the procedure of Muthukkaruppan et al., Science, Vol. 205, 1416-18 (1979) and treatment was begun 24 hours later. Heparin (oral)-cortisone acetate tapered dosage significantly inhibited capillary growth (0.02 mm/day) compared to cortisone acetate alone (0.24 mm/day), heparin alone (0.32 mm/day) or saline (0.23 mm/day). In the presence of heparin-cortisone acetate, a thin plate of tumor remained avascular. Three-dimensional tumor-growth did not occur. In contrast, in the saline controls or when either heparin or cortisone acetate were administered alone, tumors became vascularized and grew as a three-dimensional mass until they eventually perforated the cornea. These large tumors could be regressed to the flat, thin intracorneal phase by the resumption of the heparin-cortisone acetate combination. However, the intracorneal tumor cells could not be eradicated; discontinuation of the heparin-cortisone acetate led to recurrence of a vascularized tumor.

Lung metastases were counted in all animals that died. A ×6 stereoscope was used. In all control animals, the lungs were heavily studded with metastases from the three types of metastasizing tumors. In contrast, when any combination of heparin+cortisone acetate was used, no metastases were found in mice bearing ovarian sarcoma; only 1 metastasis was found in a mouse bearing Lewis lung carcinoma; and 2

6

avascular metastases less than 0.1 mm diameter were found in one mouse bearing B-16 melanoma. The nearly complete absence of metastases in heparin+cortisone treated mice was no striking, that the effect can be more readily appreciated by the following expression of data:

Total number lung metastases
Controls           =   4553 in 73 animals
Heparin+Cortisone =   3 in 39 animals

Furthermore, no lung metastases appeared in any surviving animals that were off treatment.

To exclude the possibility that tumor regression might be caused by direct cytotoxicity, all 4 types of tumor cells were cultured in the presence of 10% serum obtained from mice receiving either heparin, cortisone acetate, heparin-cortisone acetate, or no drug. Heparin-cortisone acetate did not inhibit cell growth, but in fact stimulated it. Furthermore, histological sections showed no evidence of a cytotoxic effect on bone marrow or intestinal mucosa in animals receiving heparin-cortisone acetate.

To exclude the possibility that heparin-cortisone acetate might induce tumor regression by promoting an immune reaction, mice were inoculated with fresh tumor cells at various intervals after they were off treatment. These tumors grew at the same rate as the original implants. Furthermore, if tumor regression was nearly complete and heparin-cortisone acetate was discontinued prematurely, the original tumor resumed its growth. Finally, by stopping and starting treatment, or by using sub-optimal doses of heparin-cortisone, bladder tumors could be maintained at nearly a constant small size (i.e., 45 to 70 mm$^3$) for periods of more than 8 weeks.

Inflammatory angiogenesis induced by implantation of silica particles into the rabbit cornea, immune angiogenesis induced by implantation of lymph node from a different rabbit, were also completely prevented by the cortisone-heparin pellets. Cortisone by itself temporarily delayed the onset of both types of angiogenesis (compared to an empty pellet), and heparin by itself delayed the onset of immune angiogenesis, but neither alone prevented angiogenesis for an extended period of time as did the corrisone-heparin combination.

Example 8

A human colon carcinoma was inoculated subcutaneously into nude [athymic] mice, and allowed to grow to a volume of 0.5 cm$^3$. Controls and treated animals received the same compounds, except that heparin in the drinking water was 1000 U/ml. also an additional treatment group included oral hydrocortisone (0.45 mg/ml) and oral heparin. Animals were housed in cages protected by millipore filter. Tumors grew progressively in all control animals, but regressed in animals treated with heparin and cortisone or heparin and hydrocortisone. The treated tumors were barely palpable after 6 weeks of therapy.

Example 9

CD-1 "Swiss" mice were used because this strain breeds easily and the fertilized females almost always conceive and subsequently deliver a full litter.

One male was left alone in the cage for 24 hours. His bedding was then changed and two females were added at 5 p.m. The females were then checked at 8 a.m. the next morning for the presence of a cervical plug, which indicates insemination. Sufficient male-female cages were set up to obtain at least 20 pregnant females.

Treatment of the inseminated females was started on the day after insemination by offering drinking water to each of four groups, as follows:

Group I — Heparin (Hepar) 1,000 U/ml in water
       II — Hydrocortisone phosphate, 0.45 mg/ml in water
       III — (I) and (II) together in water
       IV — Water alone

The treatments were continued for only four days, after which female mice were placed one per cage, and followed closely for the presence of offspring. The cages were checked daily for any sign of abortion (for, fetal remains, etc.).

Groups I, II and IV all produced healthy litters. In Group III there were no mice born and no evidence of abortion. This supports the conclusion that anti-angiogenesis by heparin-cortisone inhibits implantation, presumably by inhibiting capillary growth from the uterus.

**Claims**

1. A composition suitable for oral or subcutaneous administration, for inhibition of angiogenesis in mammals, in which the active agents consist essentially of 1) heparin or a heparin fragment which is a hexasaccharide or larger and 2) cortisone or hydrocortisone or the 11-alpha isomer of hydrocortisone.

2. A composition as claimed in Claim 1 in which the active agents consist essentially of heparin and hydrocortisone.

7

**0 114 589**

3. A composition as claimed in Claim 1 in which · the active agents consist essentially of a hexasaccharide heparin fragment and cortisone.

4. A composition as claimed in Claim 1 in which the active agents consist essentially of a hexasaccharide heparin fragment and hydrocortisone.

5. A composition as claimed in Claim 1 in which the active agents consist essentially of a hexasaccharide heparin fragment and hydrocortisone, and including an antibiotic.

6. A process for preparing a composition according to any of claims 1 to 5, which comprises mixing the active ingredients, either dry or in a suitable carrier.

7. A process according to claim 6 in which the active ingredients are dissolved or suspended in a non-toxic and physiologically acceptable carrier.


**Patentansprüche**

1. Zusammensetzung, die zur oralen oder subkutanen Verabreichung geeignet ist, zum Inhibieren von Angiogenese in Säugern, in der die Wirkstoffe im wesentlichen aus 1) Heparin oder einem Heparinfragment, das ein Hexasaccharid oder größer ist, und 2) Cortison oder Hydrocortison oder dem 11α-Isomer von Hydrocortison bestehen.

2. Zusammensetzung, wie in Anspruch 1 beansprucht, in der die Wirkstoffe im wesentlichen aus Heparin und Hydrocortison bestehen.

3. Zusammensetzung, wie in Anspruch 1 beansprucht, in der die Wirkstoffe im wesentlichen aus einem Hexasaccharidheparinfragment und Cortison bestehen.

4. Zusammensetzung, wie in Anspruch 1 beansprucht, in der die Wirkstoffe im wesentlichen aus einem Hexasaccharidheparinfragment und Hydrocortison bestehen.

5. Zusammensetzung, wie in Anspruch 1 beansprucht, in der die Wirkstoffs im wesentlichen aus einem Hexasaccharidheparinfragment und Hydrocortison einschließlich einem Antibiotikum bestehen.

6. Verfahren zum Herstellen einer Zusammensetzung nach einem der Ansprüche 1 bis 5, welches das Mischen der aktiven Bestandteile, entweder trocken oder in einem geeigneten Träger, umfaßt.

7. Verfahren nach Anspruch 6, in dem die aktiven Bestandteile in einem nicht-toxischen und physiologisch annehmbaren Träger gelöst oder suspendiert werden.


**Revendications**

1. Composition convenant pour l'administration orale ou sous-cutanée, pour l'inhibition de l'angiogenése chez les mammiféres, dans laquelle les agents actifs se composent essentiellement 1) d'héparine ou d'un fragment d'héparine qui est un hexasaccharide ou un saccharide supérieur et 2) de cortisone ou d'hydrocortisone ou de l'isomère 11-α de l'hydrocortisone.

2. Composition suivant la revendication 1, dans laquelle les agents actifs se composant essentiellement d'héparine et d'hydrocortisone.

3. Composition suivant la revendication 1, dans laquelle les agents actifs se composent essentiellement d'un fragment d'héparine hexasaccharide et de cortisone.

4. Composition suivant la revendication 1, dans laquelle les agents · actifs se composent essentiellement d'un fragment d'héparine hexasaccharide et d'hydrocortisone.

5. Composition suivant la revendication 1, dans laquelle les agents actifs se composent essentiellement d'un fragment d'héparine hexasaccharide et d'hydrocortisone, et contenant un antibiotique.

6. Procédé pour préparer une composition suivant l'une quelconque des revendications 1 à 5, comprenant le mélange des ingrédients actifs, soit secs, soit dans un support approprié.

7. Procédé suivant la revendication 6 dans lequel les ingrédients actifs sont dissous ou mis en suspension dans un support non toxique et physiologiquement acceptable.